(19) Europäisches Patentamt
European Patent Office
Office européen des brevets

(11) Publication number: 0 126 344 A2

# (12) EUROPEAN PATENT APPLICATION

(21) Application number: **84104919.0**

(22) Date of filing: **02.05.84**

(51) Int. Cl.³: **C 07 C 103/52**, A 61 K 37/02

(30) Priority: **20.05.83 US 496379**

(43) Date of publication of application: **28.11.84 Bulletin 84/48**

(84) Designated Contracting States: **BE CH DE FR GB IT LI NL SE**

(71) Applicant: **ABBOTT LABORATORIES, 14th Street and Sheridan Road, North Chicago, Illinois 60064 (US)**

(72) Inventor: **Frieberg, Leslie Alan, 10066 W. Hendee, Waukegan Illinois 60087 (US)**
Inventor: **Thomas, Alford Mitchell, 38660 Shagbark Lane, Wadsworth Illinois 60083 (US)**
Inventor: **Marcotte, Patrick Allen, 1840 North Delany Road, Apt. 415, Gurnee Illinois 60031 (US)**

(74) Representative: **Modiano, Guido et al, MODIANO, JOSIF, PISANTY & STAUB Modiano & Associati Via Meravigli, 16, I-20123 Milan (IT)**

(54) **Tripeptide esters of therapeutic agents.**

(57) Disclosed herein are tripeptidyl esters of the formula:

D–P

wherein D is the residue of a hydroxyl-containing therapeutic agent, and P is an N-terminal tripeptidyl moiety having the amino acid residue sequence:

$-AA_1-AA_2-AA_3$

wherein $AA_1$ is a secondary amino acid residue, $AA_2$ is a primary amino acid residue and $AA_3$ is an N-terminal amino acid residue capable of acting as an aminopeptidase substrate, and the pharmaceutically acceptable salts thereof. The tripeptidyl esters have been found to release the therapeutic agent in the presence of aminopeptidase.

EP 0 126 344 A2

Background Of The Invention

This invention relates to certain tripeptide esters of therapeutic agents, to pharmaceutical compositions containing tripeptide esters of therapeutic agents and to the therapeutic uses of such esters.

Many therapeutic agents exhibit physical properties upon *in vivo* administration which detract from their overall desireability. As one illustrative example, the macrolide antibiotics have been found to exhibit highly effective antibacterial action against such organisms as *Staphylococcus aureus*, group-A streptococci, *Streptococcus pneumoniae*, *Mycoplasma pneumoniae*, *Hemophilus influenzae*, *Treponema pallidum*, *Corynebacterium diphtheriae*, *Corynebacterium minutissimum*, *Entamoeba histolytica*, *Listeria monocytogenes*, and *Bordetella pertussis*. These compounds are therefore highly useful, *inter alia* in the treatment of susceptible upper and lower respiratory tract, skin and soft tissue infections. Despite their high level of utility as antibiotic therapeutic agents, the macrolides have been known in some cases to exhibit irregular oral bioavailability, undesireable gastrointestinal side effects, such as abdominal cramping and discomfort, and moderate to severe pain upon injection. Thus, the search continues for modified therapeutic agents, such as modified macrolide antibiotics and others, which exhibit the desired therapeutic effects of the parent therapeutic agent while reducing or eliminating undesireable physical characteristics or side effects of the therapeutic agent upon *in vivo* administration.

It has now been found that certain tripeptidyl esters of therapeutic agents are capable of being converted to the parent therapeutic agent in the

presence of aminopeptidase, such as upon *in vivo* administration to an aminopeptidase-containing subject.

## Detailed Description Of The Invention

The compounds of the present invention are tripeptide esters of therapeutic agents, which may be represented by the formula

$$D\text{-}P \qquad I.$$

wherein D is the residue of a hydroxyl-containing therapeutic agent and P is an N-terminal tripeptidyl moiety having the amino acid residue sequence

$$-AA_1-AA_2-AA_3$$

wherein $AA_1$ is a secondary amino acid residue, $AA_2$ is a primary amino acid residue and $AA_3$ is an N-terminus amino acid residue capable of acting as an aminopeptidase substrate, and the pharmaceutically acceptable salts thereof.

As used herein, the term secondary amino acid residue means the residue of an amino acid having a secondary α-amino group. Examples of naturally occurring secondary amino acid residues useful in the practice of the invention include L-prolyl, L-4-hydroxyprolyl and sarcosyl. Other useful secondary amino acid residues may be formed by substitution of a primary α-amino acid, e.g., by *N*-alkylation to form an α-amino group of the formula

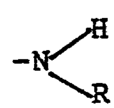

wherein R is an alkyl group having from 1 to 16 carbon atoms, preferably from 1 to 8 carbon atoms, such as L-*N*-methylalanyl, L-*N*-methylhistidyl, L-*N*-ethylglycyl, L-*N*-ethylalanyl, L-*N*-ethylhistidyl, or the like.

The term primary amino acid residue means the residue of an amino acid having a primary α-amino

group. Examples of useful primary amino acid residues include glycyl, L-alanyl, 2-aminoisobutyric acid, L-histidyl, L-asparaginyl, L-cysteinyl, L-cystinyl, L-3,5-dibromotyrosyl, L-3,5-diiodotyrosyl, L-isoleucyl, L-leucyl, L-lysyl, L-methionyl, L-phenylalanyl, L-seryl, L-threonyl, L-thyroxyl, L-tryptophyl, L-tyrosyl, L-valyl, and the like.

Suitable N-terminus amino acid residues capable of acting as an aminopeptidase substrate can be any $\alpha$-amino acid residue which is subject to cleavage from the tripeptidyl moiety due to the action of aminopeptidase enzymes. Aminopeptidase enzymes, such as leucine aminopeptidase, aminopeptidase A and the like, occur widely in nature and are commonly found in the blood serum and tissues of all mammalian species. Examples of N-terminus amino acid residues suitable for use in connection with the practice of the invention include L-leucyl, L-phenylalanyl, L-valyl, L-alanyl, L-lysyl, glycyl, L-seryl, L-aspartyl, L-glutaminyl, L-asparaginyl, L-arginyl and the like.

Therapeutic agents useful in the compounds of the invention can be any therapeutically useful, hydroxyl-containing moieties capable of being esterified with an N-terminal tripeptide of the invention. For purposes of illustration, the therapeutic agents will hereinafter be exemplified by the erythromycin moiety, although it is contemplated that other therapeutic agent moieties will be equally useful in the practice of the inventive concepts. For example, tripeptide esters of the invention may be formed with other illustrative therapeutic agent moieties such as other hydroxyl-containing macrolide antibiotics, e.g., leucomycins $A_1$ and $A_3$, and the like; aminoglycoside antibiotics, e.g., gentamicin, fortimicin A, 3-o-dimethylfortimicin A, sagamicin and the like;

steroids, e.g., aldosterone, hydrocortisone and the like; cardiovascular agents, e.g., phentolamine, propanolol and the like; cytotoxic agents and many others. As can be seen by the foregoing, it is contemplated that the tripeptidyl esters of the invention are widely applicable to hydroxyl-containing therapeutic agents in general. When the compounds of the invention are tripeptidyl esters of erythromycin compounds, or pharmaceutically acceptable salts thereof, the compounds may be used in the treatment of an infection caused by a susceptible organism by administering to an aminopeptidase-containing subject an antibacterially effective amount of said compounds. When the compounds of the invention are tripeptidyl esters of other therapeutic agents, the compounds may be used in the presence of aminopeptidase to obtain the normal purpose of such other therapeutic agents.

The term "pharmaceutically acceptable salts", as used herein, refers to the nontoxic acid addition salts of the compounds of this invention. These salts can be prepared *in situ* during the final isolation and purification of the compounds of formula I, or by separately reacting the free base with a suitable organic or inorganic acid. Representative salts include the hydrochloride, hydrobromide, sulfate, bisulfate, acetate, oxalate, valerate, oleate, palmitate, stearate, laurate, borate, benzoate, lactate, phosphate, tosylate, citrate, maleate, fumarate, succinate, tartrate, napsylate, glucoheptonate, lactobionate, lauryl sulfate and the like. It will be apparent to those skilled in the art that, depending upon the number of available amino groups for salt formation, the salts of this invention can be per-*N*-salts.

It has been found that in the presence of aminopeptidase, the presently preferred tripeptide

esters of the invention release the free therapeutic agent. The compounds of the invention are therefore useful as prodrugs which exhibit the activity of the therapeutic agent when administered to an aminopeptidase-containing subject. While the precise mechanism of therapeutic agent release has not been fully demonstrated, it is presently believed that dipeptide esters of the formula

$$D\text{-}AA_1\text{-}AA_2$$

wherein D is the residue of a hydroxyl-containing therapeutic agent moiety, $AA_1$ is a secondary amino acid residue and $AA_2$ is a primary amino acid residue, spontaneously fragment in aqueous solution or biological fluids to yield the parent therapeutic agent and a diketopiperazine. However, when the terminal amino group of the dipeptidyl ester is blocked by a third amino acid residue, no such fragmentation can spontaneously occur and the tripeptitidyl ester remains stable in aqueous solution or non-aminopeptidase-containing biological fluids. When the terminal amino acid residue ($AA_3$) is removed from the tripeptidyl ester through aminopeptidase activity, the secondary amino acid residue ($AA_1$) and the primary amino acid residue ($AA_2$) spontaneously fragment in aqueous solution or biological fluids to release the parent therapeutic agent.

The compounds of the invention may be formed by reacting the acid chloride of a N-protected secondary amino acid with a hydroxyl-containing therapeutic agent to obtain the corresponding N-protected monopeptidyl ester. Conventional amine protecting groups, such as the carbobenzyloxy-group (CBZ) or other amine protecting groups which are well known in the art, may be employed

for this purpose. The monopeptidyl-ester is then hydrogenated to remove the amine protecting group and reacted with a suitable N-protected dipeptide in the presence of a coupling agent, such as dicyclohexylcarbodiimide, to obtain the corresponding N-protected tripeptidyl ester. Upon removal of the protecting group, the desired tripeptidyl ester is obtained.

When the therapeutic agent residue is derived from a compound having more than one hydroxyl group, such as the antibiotic compound erythromycin A, a tripeptidyl ester of the invention may be formed at more than one position in the therapeutic agent molecule. Thus, erythromycin A-based compounds of the invention include the 2'-tripeptide esters, 4"-tripeptide esters, 11-tripeptide esters, 2',4"-bis(tripeptide) esters, 4",11-bis(tripeptide)esters and 2',4",11-tris(tripeptide) esters of erythromycin A. These compounds can be represented by the formulae

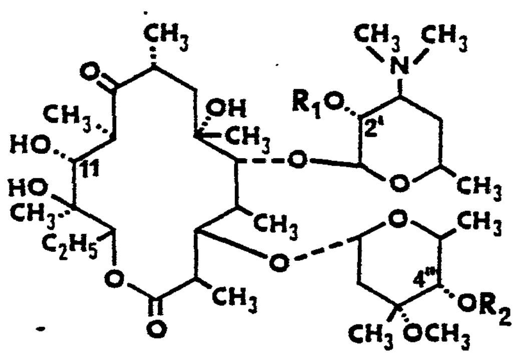

II

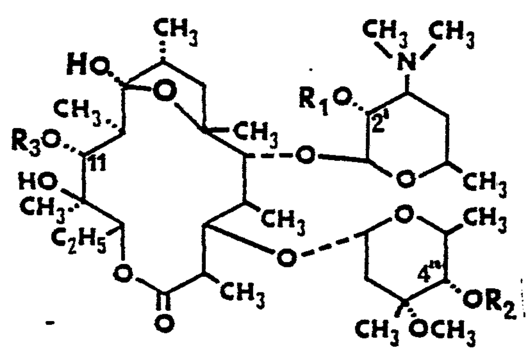

III

wherein at least one of $R_1$ or $R_2$ in formula II and $R_3$ in formula III is an N-terminal tripeptidyl moiety having the amino acid residue sequence

$$-AA_1-AA_2-AA_3$$

wherein $AA_1$ is a secondary amino acid residue, $AA_2$ is a primary amino acid residue, and $AA_3$ is an N-terminus amino acid residue capable of acting as an aminopeptidase substrate, and the remaining of $R_1$ or $R_2$ is hydrogen. The tripeptiyl esters of the invention further include mono- or di-tripeptidyl esters of the known 2'-esters of erythromicin A having therapeutic activity, such as erythromycin 2'-propanoate (erythromycin estolate), erythromycin 2'-ethylsuccinate and the like.

The foregoing may be better understood in connection with the following illustrative examples, in which amino acid residues are abbreviated as shown in Table I:

| Amino Acid Residue | Abbreviation |
|---|---|
| alanyl | ala |
| 2-aminoisobutyryl | aibu |
| arginyl | arg |
| asparaginyl | asp($NH_2$) |
| aspartyl | asp |
| cysteinyl | cySH |
| cystinyl | cyS-Scy |
| glutamoyl acid | glu |
| glutaminyl | glu($NH_2$) |
| glycyl | gly |
| histidyl | his |
| hydroxylysyl | hylys |
| hydroxyprolyl | hypro |
| isoleucyl | ileu |
| leucyl | leu |
| lysyl | lys |
| methionyl | met |
| phenylalanyl | phe |
| prolyl | pro |
| sarcosyl | sar |

| | |
|---|---|
| seryl | ser |
| threonyl | thr |
| thyroxyl | thy |
| tryptophyl | try |
| tyrosyl | tyr |
| valyl | val |

## Example 1

## 2'-Acetyl-4"-carbobenzyloxy-L-pro-erythromycin A

A suspension was formed of 40 g. of carbobenzyloxy-L-proline in anhydrous ethyl ether. To this suspension was added 35.2g. of phosphorous pentachloride, and the resulting mixture was allowed to react for 1 hour at ambient temperature. The ether solvent and phosphorus oxychloride byproducts were removed by evaporation *in vacuo* to leave the acid chloride of carbobenzyloxy-L-proline as a viscous oil. To the acid chloride of carbobenzyloxy-L-proline in 400 ml. of anhydrous ethyl ether was added 20 g. of 2'-acetylerythromycin A in 400 ml. of pyridine. The resulting reaction mixture was allowed to react for 1 hour at 0° and then partitioned between ethyl acetate and aqueous potassium carbonate. The organic layer was then separated and dried to yield 21.2 g. of a yellowish solid. The solid was crystallized from cold isopropyl alcohol to obtain 2'-acetyl-4"-carbobenzyloxy-L-pro-erythromycin A, M.P. 135-142°C.

Anal. calcd. for $C_{53}H_{86}N_4O_{16}$
C, 62.01; H, 8.21; N, 2.78
Found: C, 62.10; H, 8.19; N, 2.62

## Example 2

## 2'-Acetyl-4"-L-pro-erythromycin A

A solution was formed of 20 g. of 2'-acetyl-4"-carbobenzyloxy-L-pro-erythromycin A in 750 ml. of warm isopropyl alcohol. To this solution was added 10 g. of 10% palladium-on-carbon catalyst, and the solution was

agitated under 3 atmospheres of hydrogen for 24 hours at ambient temperature. The solution was then filtered and the filtrate evaporated *in vacuo* to yield 14.2 g. of a solid. The solid was crystallized from cold isopropyl alcohol to yield 2'-acetyl-4"-L-pro-erythromycin A, M.P. 137-157°C.

Anal. calcd. for $C_{44}H_{76}N_2O_{15}$:
C, 60.53; H, 8.77; N, 3.21
Found: C, 60.40; H, 8.70; N, 3.01

## Example 3

### 2'-Acetyl-4"-carbobenzyloxy-L-leu-gly-L-pro-erythromycin A

A reaction mixture was formed of 4.65 g. of carbobenzyloxy-L-leucylglycine, 3.0 g. of dicyclohexylcarbodiimide and 10.5 g. of 2'-acetyl-4"-L-pro-erythromycin A in 180 ml. of dry acetonitrile. The reaction mixture was allowed to react for 1 hour at ambient temperature. The mixture was then filtered and the filtrate concentrated *in vacuo*. The residue was then partitioned between ethyl acetate and aqueous potassium carbonate, and the organic layer was dried and evaporated *in vacuo* to form a second residue. The second residue was digested with petroleum ether, filtered and dried to yield 10 g. of 2'-acetyl-4"-carbobenzyloxy-L-leu-gly-L-pro-erythromycin A as a glassy solid.

## Example 4

### 4"-Carbobenzyloxy-L-leu-gly-L-pro-erythromycin A

A solution was formed of 10 g. of 2'-acetyl-4"-carbobenzyloxy-L-leu-gly-L-pro-erythromycin A in 150 ml. of methanol, and was allowed to stand at ambient temperature for 72 hours. The solvent was then evaporated *in vacuo* and the residue was triturated with

petroleum ether to yield 9 g. of 4"-carbobenzyloxy-L-leu-gly-L-pro-erythromycin A.

Example 5

4"-L-Leu-gly-L-pro-erythromycin A

To a solution of 9 g. of 4"-carbobenzyloxy-L-leu-gly-L-pro-erythromycin A in 1000 ml. of methanol was added 2.5 g. of 20% palladium-on-carbon catalyst. The solution was agitated under 3 atmospheres of hydrogen for 1 hour at ambient temperature. The solution was then filtered and evaporated *in vacuo*, and the residue was crystallized from methanol to yield 5.2 g. of 4"-L-leu-gly-L-pro-erythromycin A, M.P. 144-150°C.

Anal. calcd. for $C_{50}H_{88}N_4O_{16}$
C, 59.98; H, 8.86; N, 5.60
Found: C, 59.65; H, 8.89; N, 5.47

Example 6

2'-Acetyl-4"-CBZ-L-val-gly-pro-erythromycin A

A solution of 3.5 g. of 2'-acetyl-4"-L-pro-erythromycin A, 1.48 g. of carbobenzyloxy-L-valylglycine and 1.0 g. of dicyclohexylcarbodiimide in 60 ml. of acetonitrile was stirred at room temperature for 1 hour. The precipitated by-product (dicyclohexylurea) was removed by filtration and the acetonitrile evaporated *in vacuo*. The residue was partitioned between ethyl acetate and 2% aqueous potassium carbonate. The organic phase was washed with saturated aqueous sodium chloride, dried with anhydrous magnesium sulfate, filtered, and evaporated *in vacuo* to yield 2' acetyl-4"-CBZ-L-val-gly-L-pro-erythromycin A as a glass which was used without purification.

Example 7

4"-CBZ-val-gly-L-pro-erythromycin A

The unpurified 2' acetyl-4"-CBZ-L-val-gly-L-pro-erythromycin A of Example 6 was dissolved in 50 ml. of methanol and allowed to stand at ambient temperature

for 3 days. The methanol was evaporated and the residue dissolved in acetonitrile. A small amount of insoluble material was removed by filtration and the acetonitrile was evaporated *in vacuo* to yield a glassy solid which was digested with heptane. The resulting powdery solid was filtered, washed with heptane and dried *in vacuo* to yield 3.68 g. of 4"-CBZ-L-val-gly-L-pro-erythromycin A.

## Example 8

### 4"-L-Val-gly-L-pro-erythromycin A

To a solution of 3.68 g. of 4"-CBZ-L-valyl-gly-L-pro-erythromycin A in 150 ml. of methanol was added 0.92 g. of 20% palladium on carbon catalyst. Hydrogen was admitted into the flask at 3 atmospheres pressure and the reaction flask shaken on a Parr hydrogenation apparatus for 1 hour. The catalyst was removed by filtration and the methanol removed by evaporation *in vacuo*. The residue was dissolved in a solvent mixture containing 1% triethylamine, 5% methanol and 94% acetonitrile. The solution was passed through a 2.6 x 49 cm (260 ml.) column of silica gel 60 which had been previously equilibrated with the same solvent mixture. The major product eluted between 320-600 ml. elution volume. These fractions were combined and evaporated to dryness *in vacuo*. The residue was dissolved in acetonitrile and insoluble material removed by filtration. The acetonitrile was removed by evaporation *in vacuo* and the residue dissolved in 10 ml. of methanol.

Crystallization commenced when the methanol solution was allowed to stand at -25°C. for several days. The crystalline material was recovered by filtration, washed with a small amount of cold methanol and dried *in vacuo* to yield 1.05 g. of purified 4"-L-val-gly-L-pro-erythromycin A.

Analysis cal'd. for $C_{49}H_{86}N_4O_{16}$:
C, 59.61; H, 8.78; N, 5.68
Found C, 60.15; H, 9.11; N, 5.40

Example 9

2'-Acetyl-4"-CBZ-L-phe-L-pro-erythromycin A

A solution of 3.59 g. of 2'-acetyl-4"-L-pro-erythromycin A, 1.71 g. of carbobenzyloxy-L-phenylalanylglycine, and 1.0 g. dicyclohexylcarbodimide was stirred at room temperature for 1 hour. The precipitated by-product (dicyclohexylurea) was removed by filtration and the acetonitrile evaporated *in vacuo*. The residue was partitioned between ethyl acetate and 2% aqueous potassium carbonate. The organic phase was dried with anhydrous magnesium sulfate, filtered, and evaporated to yield 2'-acetyl-4"-CBZ-L-phe-gly-L-pro-erythromycin A as a glass which was used without purification.

Example 10

4"-CBZ-L-phe-gly-pro-erythromycin A

The unpurified 2'-acetyl-4"-CBZ-L-phe-gly-L-pro-erythromycin A of Example 9 was dissolved in 50 ml. of methanol and allowed to stand at room temperature for 3 days. The methanol was evaporated and the residue dissolved in acetonitrile. A small amount of insoluble material was removed by filtration and the acetonitrile was evaporated *in vacuo*. The glassy residue was digested with heptane to yield a powdery solid which was filtered, washed with heptane and dried *in vacuo* to yield 4.23 g. of 4"-CBZ-L-phe-gly-L-pro-erythromycin A.

Example 11

4"-L-Phe-gly-L-pro-erythromycin A

To a solution of 4.23 g. of 4"-CBZ-L-phe-gly-L-pro-erythromycin A in 250 ml. of methanol was added 1.2 g. of 20% palladium on carbon catalyst. Hydrogen was admitted into the flask at 3 atmospheres pressure and

the reaction flask shaken on a Parr hydrogenation apparatus for 1 hour. The catalyst was removed by filtration and the methanol solution evaporated in vacuo.

The residue was dissolved in a solvent mixture containing 1% triethylamine, 5% methanol and 94% acetonitrile. The solution was passed through a 2.6 x 49 cm (260 ml.) column of silica gel 60 which had been previously equilibrated with the same solvent mixture. The major product elutes between 360-720 ml. elution volume. These fractions were combined and evaporated to dryness. The residue was dissolved in acetonitrile and a small amount of insoluble material removed by filtration. The acetonitrile was then removed by evaporation in vacuo.

After attempting unsuccessfully to crystallize the residue from methanol or ethyl ether, the crude product was digested with heptane to yield 2.6 g. of a powdery solid which was recovered by filtration and dried. The solid material (2.0 g.) was crystallized from ethanol to obtain 0.84 g. of purified 4"-L-phe-gly-L-pro-erythromycin A.

Anal. calcd. for $C_{53}H_{86}N_4O_{16}$:
C, 61.49; H, 8.38; N, 5.41
Found C, 61.23; H, 8.24; N, 5.17

## Example 12

## 2'-Acetyl-4"-CBZ-L-ala-gly-L-pro-erythromycin A

A solution of 3.5 g. 2'-acetyl-4"-L-pro-erythromycin A, 1.34 g. of carbobenzyloxy-L-alanyl glycine and 1.0 g. dicyclohexylcarbodiimide in 60 ml. acetonitrile was stirred at room temperature for 1 hour. The preciptated by product (dicyclohexylurea) was removed by filtration and the acetonitrile was evaporated in vacuo. The residue was partitioned between ethylacetate and 2% aqueous potassium carbonate. The organic phase was washed with saturated

aqueous sodium chloride, dried with anhydrous magnesium sulfate, filtered and evaporated to yield 2'-acetyl-4"-CBZ-L-ala-gly-L-pro-erythromycin A as a glass which was used without purification.

Example 13

4"-CBZ-L-Ala-gly-L-pro-erythromycin A

The unpurified 2'-acetyl-4"-CBZ-L-ala-gly-L-pro-erythromycin A of Example 12 was dissolved in 50 ml. methanol and allowed to stand at ambient temperature for 3 days. The methanol was evaporated and the residue dissolved in acetonitrile. A small amount of insoluble material was removed by filtration and the acetonitrile was evaporated *in vacuo* to yield a glassy solid which was digested with heptane. The powdery solid was filtered, washed with heptane and dried *in vacuo* to yield 3.76 g. of 4"-CBZ-L-ala-gly-L-pro-erythromycin A.

Example 14

4"-L-Ala-gly-L-pro-erythromycin A

To a solution of 3.76 g. of 4"-CBZ-L-ala-gly-L-pro-erythromycin in 500 ml. of methanol was added 1.0 g. of 20% palladium on carbon catalyst. Hydrogen was admitted into the flask at 3 atmospheres pressure and the reaction flask shaken on a Parr hydrogenation apparatus for 1 hour. The catalyst was removed by filtration and the methanol removed by evaporation *in vacuo*. The residue was dissolved in a solvent mixture containing 1% triethylamine, 10% methanol, 89% acetonitrile. The solution was passed through a 3.0 x 60 cm (424 ml.) column of silica gel 60 which had been previously equilibrated with the same solvent mixture. The major product elutes between 680-1200 ml. elution volume. These fractions were combined and evaporated to dryness. The residue was dissolved in acetonitrile and a small amount of insoluble material removed by

filtration. The acetonitrile was removed by evaporation *in vacuo* and the residue dissolved in about 2.5 ml. of methanol. The desired product, 4"-L-ala-gly-L-pro-erythromycin A, crystallizes from methanol at -25°C., but the cold solvent dissolves much of the product. The crystals are quickly filtered, washed with a small amount of cold methanol and dried to yield 0.86 g. of purified 4"-L-ala-gly-L-pro-erythromcyin A.

Anal. calcd. for $C_{47}H_{82}N_6O_{16}$:
C, 58.85; H, 8.62; N, 5.84
Found C, 58.42; H, 8.72; N, 5.75

Example 15

2'-Acetyl-4"-carbobenzyloxysarcosyl-erythromycin A

A suspension was formed of 12.25 g. of the sodium salt of carbobenzyloxysarcosine in 250 ml. of toluene and the suspension was cooled to -78°C. To the cooled suspension was added 4.0 ml. of oxalyl chloride. After 90 minutes at -78°C, 5 g. of 2'-acetyl-erythromycin A in 100 ml. of pyridine was added to the suspension to form a reaction mixture. The mixture was stirred for 90 minutes at -78°C. and then for 2 hours at -20°C. The reaction mixture was then evaporated *in vacuo* and the residue partitioned between ethyl acetate and aqueous potassium carbonate. The organic phase was dried and evaporated *in vacuo* and the residue chromatographed on a silica gel column, eluted with 0.5% methanol, 0.5% triethylamine and 99% acetonitrile to yield 2.3 g. of 2'-acetyl-4"-carbobenzyloxysarcosyl-erythromycin A as a yellowish solid.

Example 16

2'-Acetyl-4"-sar-erythromycin A

A solution was formed of 2.0 g. of 2'-acetyl-4"-carbobenzyloxysarcosyl-erythromycin A in 75 ml. of isopropyl alcohol. To the solution was added 1 g. of 10% palladium-on-carbon catalyst, and the

solution was agitated under 3 atmospheres of hydrogen for 24 hours at ambient temperature. The solution was then filtered and the filtrate evaporated <u>in vacuo</u> to yield 1.4 g. of 2'-acetyl-4"-sar-erythromycin A as an off-white solid. A sample of the product was crystallized from benzene to obtain 2'-acetyl-4"-sar-erythromycin A, m.p. 129-133 C°.

Anal. calcd. for $C_{42}H_{74}N_2O_{15}$:
C, 59.56; H, 8.81; N, 3.31
Found C, 59.51; H, 8.66; N, 3.67

Example 17

4"-L-Leu-gly-sar-erythromycin A

The procedure of Examples 3-5 was followed replacing the 2'-acetyl-4"-L-pro-erythromycin A in Example 3 with 2'-acetyl-4"-sar-erythromycin A to obtain 4"-L-leu-gly-sar-erythromycin A. A sample of the product was crystallized from methanol at -25° C to obtain 4"-L-leu-gly-sar-erythromycin A, m.p. 126-134° C.

Example 18

2'-Acetyl-4"-L-leu-gly-L-pro-erythromycin A

A solution of the 2'-acetyl-4"-carbobenzyloxy-L-leu-gly-L-pro-erythromycin A of Example 3 in isopropanol is hydrogenated according to the procedure of Example 5 to obtain 2'-acetyl-4"-L-leu-gly-L-pro-erythromycin A.

Example 19

2'-Propionyl-4"-L-leu-gly-L-pro-ethromycin A

The procedure of Examples 1-3 is repeated replacing the 2'-acetyl-erythromycin A in Example 1 with 2'-propionyl-erythromycin A to obtain 2'-propionyl-4"-carbobenzyloxy-L-leu-gly-L-pro-erythromycin A. A solution of 2'-propionyl-4"-carbobenzyloxy-L-leu-gly-L-pro-erythromycin A in isopropanol is hydrogenated according to the procedure of Example 5 to obtain 2'-propionyl-4"-L-leu-gly-L-pro-erythromycin A.

Example 20

2'-Ethylsuccinyl-4"-L-leu-gly-L-pro-erythromycin A

The procedure of Examples 1-3 is repeated replacing the 2'-acetyl-erythromycin A of Example 1 with 2'-ethylsuccinyl-erythromycin A to obtain 2'-ethylsuccinyl-4"-carbobenzyloxy-L-leu-gly-L-pro-erythromycin A. A solution of 2'-ethylsuccinyl-4"-carbobenzyloxy-L-leu-gly-L-pro erythromycin A in isopropanol is hydrogenated according to the procedure of Example 5 to obtain 2'-ethylsuccinyl-4"-L-leu-gly-L-pro-erythromycin A.

Example 21

The general procedure of Examples 1-5 is followed replacing the carbobenzyloxy-L-proline in Example 1 with carbobenzyloxy-L-AA, and replacing the carbobenzyloxy-L-leucylglycine of Example 3 with carbobenzyloxy-L-$AA_3$-$AA_2$-H to obtain 4"-$AA_3$-$AA_2$-$AA_1$-erythromycin A wherein $AA_1$, $AA_2$ and $AA_3$ are as hereinbefore defined.

Example 22

2'-Acetyl-4"-benzyloxycarbonyl-erythromycin A

A mixture of 46.57 g. of 2'-acetyl-erythromycin A and 29.34 g. of dimethylaminopyridine was dissolved in 500 ml. of $CH_2Cl_2$ and was cooled to -25°C. Then, 25.7 ml. of benzylchloroformate was added over a period of 30 minutes. The mixture was kept at -25°C. for 4 days. The $CH_2Cl_2$ soluton was washed with two 300 ml. portions of aqueous 4% $KH_2PO_4$, and finally with 200 ml. of aqueous 2% $NaHCO_3$. The $CH_2Cl_2$ layer was dried over anhydrous $Na_2SO_4$ and the solvent was removed with a rotary evaporator. The residue (58.4 g.) was vacuum dried for 1 day and then was crystallized twice from $CH_3CN$ at -25°C. The crystals were dried at 50°C. in a vacuum oven to give 35.25 g. (65% yield) of

2'-acetyl-4"-benzyloxycarbonyl-erythromycin A, m.p. 123-128°C.

Anal. Calcd. for $C_{47}H_{75}NO_{16}$
C, 62.03; H, 8.31; N, 1.54
Found: C, 61.77; H, 8.11; N, 1.87

## Example 23

### 2'-Acetyl-4"-benzyloxycarbonyl-11-N-carbobenzyloxy-L-pro-erythromycin A 6,9-hemiketal

A mixture of 16.38 g. of 2'-acetyl-4"-benzyloxycarbonyl-erythromycin A and 2.21 g. of 4-dimethylaminopyridine was dissolved in 100 ml. of $CH_2Cl_2$. A solution was prepared from 9.4 ml. of diisopropylethylamine, 13.63 g. of N-carbobenzyloxy-L-pro, and 100 ml. of $CH_2Cl_2$ was added and the mixture was cooled to 0°C. Finally, 11.16 g. of dicyclohexylcarbodiimide, dissolved in 50 ml. of $CH_2Cl_2$ was added and the reaction mixture was kept at 0°C. for 18 hours. The reaction mixture was filtered and concentrated to a syrup using a rotary evaporator. The syrup was dissolved in 300 ml. of benzene and the benzene solution was washed with the following: two 100 ml. portions of 2% aqueous $NaHCO_3$; three 100 ml. portions of 9 to 1 2% aqueous $NaHCO_3$/concentrated ammonium hydroxide; four 100 ml. portions of pH 6.5 0.24 M phosphate buffer; and finally 100 ml. of 2% aqueous $NaHCO_3$. The benzene layer was dried over anhydrous $Na_2SO_4$ and was treated with 2 g. silica gel. The benzene was filtered and was evaporated using a rotary evaporator. The dried residue was triturated with 200 ml. of hexane. The white solid was dried in a vacuum oven to give 18.00 g. (88% yield) of 2'-acetyl-4"-benzyloxycarbonyl-11-N-carbobenzyloxy-L-pro-erythromycin A 6,9-hemiketal as a white solid. A sample crystallized from isopropanol had m.p. 161-163°C.

 

Anal. Calcd. for $C_{60}H_{88}N_2O_{19}$
C, 63.14; H, 7.77; N, 2.45
Found: C, 63.14; H, 7.69; N, 2.29

Example 24

2'-Acetyl-4"-benzyloxycarbonyl-11-N-carbobenzyloxy-sar-erythromycin A 6,9-hemiketal

A mixture of 16.38 g. of 2'-acetyl-4"-benzyloxycarbonyl-erythromycin A, 2.24 g. of 4-dimethylaminopyridine, 6.97 g. of diisopropylethylamine, and 12.06 g. of N-carbobenzyloxysarcosine in 250 ml. of $CH_2Cl_2$ was cooled at 0°C. Then, 11.17 g. of dicyclohexylcarbodiimide was added and the reaction mixture was kept at 0°C. for 18 hours. The reaction mixture was filtered and concentrated to a syrup using a rotary evaporator. The syrup was dissolved in 300 ml. of benzene and was washed using the procedure of Example 22. The benzene was dried over anhydrous $Na_2SO_4$, was filtered, and was concentrated using a rotary evaporator. The concentrated syrup produced a foamed glass under vacuum and was triturated with 200 ml. of hexane. Further purification of the yellow solid was accomplished by the following treatment. The solid was mixed with hexane: 5% $CH_2Cl_2$ forming an unsoluble yellow oil from which the hexane mixture was decanted. The oil was dissolved in 200 ml. of benzene and 5 g. of silica gel was added. After stirring several minutes the benzene was filtered and concentrated to dryness using a rotary evaporator. The product was foamed under high vacuum and was triturated with heptane to give 16.89 g. (84% yield) of 2'-acetyl-4"-benzyloxycarbonyl-11-N-carbobenzyloxy-sar-erythromycin A 6,9-hemiketal.

Anal. Calcd. for $C_{58}H_{86}N_2O_{19}$

C, 62.46; H, 7.77; N, 2.51

Found: C, 62.63; H, 7.69; N, 2.42

Example 25

11-L-Leu-gly-sar-erythromycin A 6,9-hemiketal

The procedure of Examples 2-5 is followed replacing the 2'-acetyl-4"-carbobenzyloxy-L-pro-erythromycin A in Example 3 with 2'-acetyl-4"-benzyloxycarbonyl-11-*N*-carbobenzyloxy-sar-erythromycin A 6,9-hemiketal to obtain 11-L-leu-gly-sar-erythromycin A 6,9-hemiketal.

Example 26

In Vitro Activity

The *in vitro* rate of hydrolysis of tripeptide esters of erythromycin A in the presence and in the absence of aminopeptidase was determined as follows.

A 50 µl sample of a 20 mg./ml. solution of a test tripeptide ester compound in methanol was added to 0.4 ml. of an aqueous solution of 50 mM Tris hydrochloride and 5 mM $MgCl_2$ at a pH of 8.0, to obtain a final concentration of the test compound of 2 mg./ml. The solution was heated to 37 ° C in a water bath. To the test solution was added 100 µl of a 1 mg./ml. solution of soluble kidney leucine aminopeptidase, and the progress of hydrolysis of the compound to Erythromycin A was monitored by thin layer chromatography.

In the foregoing manner, the hydrolysis rate of the following test compound was determined as shown in Table I.

Example 27

In Vivo Activity

Eight groups of ten female CF-1 mice, each weighing 18-20 g. were supplied with food and water *ad lib* and injected intraperitoneally with a lethal dose

(100 x $LD_{50}$) of *Staph. aureus*, strain 642a. At 1 and 5 hours after intraperitoneal infection, the mice were administered by intraperitoneal injection equal doses of either erythromycin A ("A") or 4"-L-leu-gly-L-pro-erythromycin A ("B") dissolved in a phosphate buffered saline solution. The number of mice surviving in each group 5 days after infection is shown in the following Table II:

Table II

| Test Compound | Total Dose (mg/kg) | Mice Surviving |
|---|---|---|
| A | 30 | 10 |
| A | 7.5 | 10 |
| A | 1.9 | 9 |
| A | 0.4 | 9 |
| B | 41.1 | 10 |
| B | 10.5 | 10 |
| B | 2.6 | 10 |
| B | 0.6 | 5 |

From the foregoing, the effective dose ($ED_{50}$) for intraperitoneal injection of erythromycin A was determined to be less than 0.4 mg./kg. and that for 4"-L-leu-gly-L-pro-erythromycin A was determined to be 0.6 mg./kg. These doseages are essentially equivalent when the increased molecular weight of the tripeptide ester is taken into consideration.

Example 28

In Vivo Activity

The procedure of Example 25 was repeated except that the erythromycin A and 4"-L-leu-gly-L-pro-erythromycin A were administered subcutaneously instead of intraperitoneally. The number of mice surviving in each group five days after infection is shown in the following Table III:

Table III

| Test Compound | Total Dose (mg/kg) | Mice Surviving |
|---|---|---|
| A | 30 | 10 |
| A | 7.5 | 10 |
| A | 1.9 | 1 |
| A | 0.4 | 1 |
| B | 41.1 | 10 |
| B | 10.5 | 6 |
| B | 2.6 | 5 |
| B | 0.6 | 2 |

From the foregoing, the effective dose ($ED_{50}$) for subcutaneous administration of erythromycin A was determined to be 2.8 mg./kg., and that for 4"-L-leu-gly-L-pro-erythromycin A was determined to be 3.1 mg./kg.

Example 29

In Vivo Activity

The procedure of Example 25 was repeated except that the erythromycin A and 4"-L-leu-gly-L-pro erythromycin A were administered orally, and that 0.5 ml of homogenized milk was administered orally to each mouse prior to administration of the test compound to minimize acid degredation of the test compound. The number of mice surviving in each group five days after infection is shown in the following Table IV:

Table IV

| Test Compound | Total Dose (mg/kg) | Mice Surviving |
|---|---|---|
| A | 150 | 7 |
| A | 37.5 | 0 |
| A | 9.4 | 0 |
| A | 2.3 | 0 |
| B | 205 | 6 |
| B | 51.3 | 0 |
| B | 12.8 | 0 |
| B | 3.2 | 0 |

From the foregoing, the effective dose ($ED_{50}$) for oral administration of erythromycin A was determined

to be 110 mg./kg. (error limits of 71 to 172 mg./kg.), and that for 4"-L-leu-gly-L-pro-erythromycin A was determined to be 181 mg./kg. (error limits of 115-285 mg./kg.).

## Example 30

## Gastrointestinal Motility

Female beagle dogs having a weight of 7.0 to 11.0 kg. were anesthetized with pentobarbital and maintained in surgical anesthesia by supplemental I.V. injections of pentobarbital as needed. The body temperature of each dog was maintained at 36-37° C. The trachea was cannulated and the animal maintained on a respirator at 12 strokes/minute with a volume of 200 ml./stroke. The femoral artery was cannulated for blood pressure recording and both femoral veins were cannulated for intravenous injections and blood collection. Each dog was then prepared for gastrointestinal motility determinations as follows. The abdomen was opened with a midline incision and strain gauge transducers, previously calibrated to 40 g. full scale, were sewn on at the following locations:

1. the stomach, 2-3 cm. orad from the pylorus;
2. the duodenum, 2-3 cm. caudad to the pylorus;
3. the jejunum, 10 cm. from the ligament of Treitz;
4. the ileum, 10 cm. orad from the ileocecal valve and on the transverse colon; and
5. the colon, 10 cm. distal to the cecum.

The abdominal cavity was packed loosely with sponges and kept moist with saline.

After an equilibration period of 15 minutes, a dose of 50 µg/.kg. of methacholine was administered intravenously to the dog over a period of thirty seconds and the strain gauge readings were monitored to obtain a

standard measurement of reactivity of the gastrointestinal tract.

The dog was then injected intravenously with 1.0 mg./kg. of either erythromycin A lactobionate or 4"-L-leu-gly-L-pro-erythromycin A in a saline or saline alcohol solution, respectively, over a ten second period, followed by 1.0 ml. of saline wash. The strain gauge readings were again monitored to determine effect of the test compound on motility of the gastrointestinal tissue. Administration of erythromycin A lactobionate resulted in pronounced stimulation of the stomach, duodenum, jejunum and ileum, with no apparent effect on the colon. Administration of 4"-L-leu-gly-L-pro-erythromycin A resulted in no apparent stimuation of the gastrointestinal tissues. The contractile or motility index was determined according to the method of Jacoby, et al., as described in "Gastrointestinal Actions of Metoclopramide", Gastroenterology, Vol. 52, No. 4 (1967), pp. 676-684 by giving a numerical score to the height of each recorded contraction in the five minute period both before and after administration of the test compound. The contractile index for the test compounds erythromycin A lactobionate and 4"-L-leu-gly-L-pro-erythromycin A is shown in the following Table V:

Table V

Contractile Index

| Tissue | Erythromycin Lactobionate | 4"-L-leu-gly-L-pro-erythromycin A |
|---|---|---|
| Stomach | 56.0 | 1.25 |
| Duodenum | 30.0 | 2.5 |
| Jejunum | 26.0 | 0.0 |
| Ileum | 13.0 | 1.0 |
| Colon | 0.0 | 0.0 |

The tripeptide esters of the invention may be administered orally or by parenteral injection, e.g., by intramuscular, intravenous, intraperitoneal or subcutaneous routes of administration.

In addition to the active compounds, compositions according to this invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain ajuvants such as preserving, wetting emulsifying, and dispersing agents. They may be sterilized, for example, by filtration through a bacteria-retaining filter, or by incorporating sterilizing agents into the compositions. They can also be manufactured in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert diluent such as sucrose, lactose or starch. Such dosage forms can also comprise, as is normal practice, additional substances other than inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixirs containing inert diluents commonly used in the art, such as water. Besides such

inert diluents, compositions can also include adjuvants, such as wetting agents, emulsifying and suspending agents, and sweetening, flavoring and perfuming agents.

Compositions for rectal administration are prefereably suppositories which may contain, in addition to the active substance, excipients such as cocoa butter or a suppository wax.

Actual dosage levels of active ingredient in the compositions of the invention may be varied so as to obtain an amount of active ingredient effective to achieve a desired therapeutic effect for a particular composition and method of administration. The selected dosage level therefore depends upon the nature of the therapeutic agent moiety, the desired therapeutic effect, the route of administration, the duration of treatment and other factors. Generally, when the therapeutic agent moiety is derived from erythromycin A, dosage levels of about 0.1 to about 1000, more preferably about 0.25 to about 750 and most preferably about 0.5 to about 500 mg. of active ingredient per kg. of body weight are administered daily to a mammalian patient suffering from an infection caused by a susceptible organism. If desired, the daily dose may be divided into multiple doses for administration, e.g., two to four times per day.

CLAIMS:

1. A compound of the formula

D-P

wherein D is the residue of a hydroxyl-containing therapeutic agent and P is an N-terminal tripeptidyl moiety having the amino acid residue sequence

$$-AA_1-AA_2-AA_3$$

wherein $AA_1$ is a secondary amino acid residue, $AA_2$ is a primary amino acid residue and $AA_3$ is an N-terminus amino acid capable of acting as an aminopeptidase substrate, or a pharmaceutically acceptable salt thereof.

2. A compound of Claim 1 wherein $AA_1$ is selected from the group consisting of L-propyl, L-hydroxypropyl, sarcosyl and an N-alkylated primary amino acid having an -amino group of the formula

$$\begin{matrix} H \\ | \\ -N-R \end{matrix}$$

wherein R is alkyl of 1 to 16 carbon atoms.

3. A compound of Claim 1 wherein $AA_2$ is selected from the group consisting of glycyl, L-alanyl, L-2-aminoisobutyryl, L-histidyl, L-asparaginyl, L-cysteinyl, L-cystinyl, L-3,5-dibromotyrosyl, L-3,5-diiodotyrosyl, L-hydroxyglycyl, L-isoleucyl, L-leucyl, L-lysyl, L-methionyl, L-phenylalanyl, L-seryl, L-threonyl, L-thyroxyl, L-tryptophyl, L-tyrosyl and L-valyl.

4. A compound of Claim 1 wherein $AA_3$ is selected from the group consisting of L-leucyl, L-phenylalanyl, L-valyl, L-lysyl, glycyl, L-seryl, L-aspartyl, L-glutamyl, L-asparaginyl and L-arginyl.

5. A compound of Claim 1 wherein D is selected from the group consisting of erythromycin A, erythromycin A 2'-propanoate and erythromycin A 2'-ethylsuccinate.

6. A pharmaceutical composition comprising a compound of Claim 1 together with a pharmaceutically acceptable carrier.

7. A pharmaceutical composition comprising a compond a Claim 5 and a pharmaceutically acceptable carrier.

8. A method of treating an aminopeptidase-containing subject comprising administering thereto an aminopeptidase-containing subject an antibacterially effective amount of a compound of Claim 5.